(19) Europäisches Patentamt
European Patent Office
Office européen des brevets

(11) **EP 1 308 725 B1**

(12) **EUROPEAN PATENT SPECIFICATION**

(45) Date of publication and mention
of the grant of the patent:
**13.10.2010 Bulletin 2010/41**

(51) Int Cl.:
*G01N 33/38* *(2006.01)*        *G01N 22/04* *(2006.01)*
*G01N 27/22* *(2006.01)*        *G01N 27/04* *(2006.01)*
*B28C 7/02* *(2006.01)*         *B28C 7/04* *(2006.01)*
*G01N 9/36* *(2006.01)*         *G01N 9/02* *(2006.01)*

(21) Application number: **02396158.4**

(22) Date of filing: **28.10.2002**

(54) **Method for moisture measurement in concrete with the help of electromagnetic fields**

Verfahren zur Messung der Feuchtigkeit in Zement mit Hilfe eines elektromagnetischen Feldes

Procédé de mesure de l'humidité du beton avec l'aide des champs électromagnétiques

(84) Designated Contracting States:
**AT BE BG CH CY CZ DE DK EE ES FI FR GB GR IE IT LI LU MC NL PT SE SK TR**

(30) Priority: **31.10.2001  FI 20012106**

(43) Date of publication of application:
**07.05.2003  Bulletin 2003/19**

(73) Proprietor: **Consolis Technology Oy Ab**
**01510 Vantaa (FI)**

(72) Inventors:
• **Käppi, Aulis**
 **21600 Parainen (FI)**
• **Henriksson, Mikko**
 **37830 Viiala (FI)**
• **Nordenswan, Erik**
 **21600 Parainen (FI)**
• **Kuosmanen, Olavi**
 **21600 Parainen (FI)**
• **Ristola, Pekko**
 **20610 Turku (FI)**

(74) Representative: **Langenskiöld, Tord Karl Walter et al**
**Oy Jalo Ant-Wuorinen Ab**
**Iso-Roobertinkatu 4-6 A**
**00120 Helsinki (FI)**

(56) References cited:
**EP-A- 1 207 384      GB-A- 2 359 138**
**US-A- 4 186 952      US-A- 5 025 954**

• **PATENT ABSTRACTS OF JAPAN vol. 1999, no. 13, 30 November 1999 (1999-11-30) & JP 11 211475 A (KUMAGAI GUMI CO LTD), 6 August 1999 (1999-08-06)**
• **PATENT ABSTRACTS OF JAPAN vol. 1996, no. 12, 26 December 1996 (1996-12-26) & JP 08 201315 A (ZENKOKU NAMA CONCRETE KOGYO KUMIAI RENGOKAI;YOKOWO CO LTD), 9 August 1996 (1996-08-09)**
• **YASUHIRO ARAI ET AL: "MEASUREMENT OF ELECTRICAL CONDUCTIVITY AND DETECTION OF WATER IN CONCRETE WITH CFGFRP USED AS ELECTRODES" JOURNAL OF THE CERAMIC SOCIETY OF JAPAN, INTERNATIONAL EDITION, FUJI TECHNOLOGY PRESS, TOKYO, JP, vol. 102, no. 8, 1 August 1994 (1994-08-01), pages 745-751, XP000495848 ISSN: 0912-9200**

EP 1 308 725 B1

**Description**

The present invention relates to a method for improving the accuracy of material moisture content measurement based on the use of electromagnetic fields, such as those employed in the microwave measurement techniques.

Moisture content measurement based on microwave gauging techniques or sensing changes in capacitance or electric conductivity is well known in the prior art. However, microwave techniques for instance cannot directly measure moisture content of a material, but instead, the dielectric properties of the material that are not only dependent on the moisture content but also on plural other factors. Consequently, the measurement of moisture content, e.g., carried over in stone aggregate used in mixing concrete, fails to give sufficiently accurate results, thus also compromising the quality control of the end product.

A crucial factor leading to inaccuracy of measurements is the volume change that occurs in small-aggregate granular material as a function of material moisture content. Granular material packs into its densest state when completely dry, whereby the aggregate particles in principle touch each other intimately. If the moisture content of the aggregate material is increased so as to wet the surfaces of the particles, the packing density of the material after working (stirring, transportation, etc.) becomes less dense than in its dry state inasmuch as it assumes a more porous structure. This is because of surface forces that appear at the liquid-air interface so as to create meniscus-like air spaces between the minute particles, thus locking the material structure into a more porous state. This kind of packaging density change due to embedded moisture is known in the art as "bulking". The bulk of aggregate structure increases with higher moisture content causing a greater number of meniscuses up to limit where the interstitial spaces between the particles begin to become filled with liquid. From this point onward, the porosity of the material structure decreases and an entirely liquid-impregnated structure again assumes a denser state. In fact, a material thoroughly impregnated by a liquid may again become densely packed.

Even having a microwave measurement performed on a wetted granular material alone, the method can only detect the number of water molecules in a given unit volume, instead of indicating the amount of water per unit weight of dry solids. On the other hand, inasmuch as the overall weight of material in a mixer is known without any information on its moisture content, the packing density of the mix remains unknown thus making it impossible to convert the results of microwave measurements directly into a moisture content value, because the amount of mineral aggregate within the effective gauging range of the microwave sensor cannot be determined. Furthermore, the response of the microwave sensor is not linear within the moisture content range of interest in practice.

The situation is further complicated if the mineral particle size of rock aggregate changes. Since water molecules adsorbed on the rock material surface or situated in a close vicinity thereto do not behave under microwave radiation in the same manner as free-water molecules at a greater distance from the surface, the microwave measurement gives an incorrect result deviating from the actual moisture content. The overall surface area of rock aggregate increases rapidly with a higher proportion of fine aggregates in the mix, whereby also the proportion of water molecules adhering to the rock material surface increases in the amount of water added to the mix along with the wet rock material.

Taking into account these factors along with the bulking effect and change in particle size, the microwave measurement method can be used for gauging the moisture content of rock material in order to determine a more exact value for the overall amount of water.

In order to utilize microwave techniques for determination of moisture content of fine aggregate per mass unit (in conjunction with metering the material into the mix by weighing), it is necessary to know, not only the number of water molecules that is the outcome of the microwave measurement, but also the mass of the unit volume where the water molecules reside, that is, the density of the material mix.

Density measurement may in principle be carried out in plural ways (e.g., performing the measurements at two different frequencies or using a gamma radiation gauge). In practice, a gamma radiation gauge is probably the most common instrument. However, it is rather expensive and necessitates user training plus professional maintenance under a permit. Also a dual-frequency microwave gauge system is more expensive and complicated than conventional microwave gauges of material moisture content used in the art today.

In GB 21 359 138 A, a method and an apparatus for monitoring moisture content and in particular for monitoring moisture content in hardening and formed concrete and in concrete aggregates ready for mixing. The moisture content is measured by means of a capacitance measuring method. The accuracy of such a method is not sufficient for the manufacturing and for controlling the manufacturing of fresh concrete. Said document does disclose any measures to improve the accuracy in measuring moisture content and the bulking effect is not disclosed at all. The only reference to change in volume due to moisture refers to the swelling effect of the alkali-silicate reaction, which is a chemical reaction, and has nothing to do with the bulking effect, which is an effect caused by meniscuses and surface forces due to embedded water.

The only application referring to the manufacture of concrete in said document is monitoring adsorbed water on concrete aggregate ready for mixing. In said application one of the electrodes needed in the capacitive measuring should be located centrally in a bulk sand dispensing hopper whereby the sidewall of the hopper will form the other electrode. Such an application cannot be used in measuring moisture content of fresh concrete in a mixer having rotating mixer elements or alternatively stationary mixer elements and a rotating mixing vessel. The electrodes needed for the capacity measurement in the mixer would be damaged or they would not give applicable measuring results.

In US-A- 5 025 954 a system is disclosed for individually measuring and dispensing components of a multi-component mixture, wherein a fluid level is monitored by ultrasonic means. However, said document does not disclose gauging the level of a granular material. Said document discloses a method wherein fluid pigments are combined to make a particular color for use in coloring concrete. The monitoring or gauging of each component is carried out in a separate measuring container before it is fed to a mixer. No level gauging is carried out in the mixer.

It is an object of the present invention to improve the measurement accuracy of microwave gauging techniques through compensating for the errors of microwave gauging techniques caused by variations in the bulk density of the material due to vary-ing material moisture content, cement paste rheology, additives, fineness modulus and type of aggregate surface area by virtue of measuring the level of concrete mix in a mixer with the help of level gauge based on ultrasound, laser or the like technique.

In addition to level gauging, the invention may utilize in the correction of moisture content measurements other known parametric correction factors of microwave measurement results, such as temperature and mix composition data (e.g., proportions of batch components specified for the mix, actual amounts of the batch components metered, properties of the different batch components, etc.).

The moisture content in the mix can be solved using, e.g., the following linear regression equation:

$$M = a + b_1R_1m_1 + b_2R_2m_2 + \ldots + b_QQ + b_TT, \qquad (1),$$

where

R = a material property of a component in the mix

m = mass of the component in the mix

Q = a dielectric property of the mix determined by microwave measurement

T = temperature

By virtue of complementing the above equation with a term $b_LL$, where L is the result of level gauging in the mixer and $b_L$ is an empirically determined constant, it has been found that the accuracy of moisture content measurement can be improved substantially.

Thus, unexpectedly good results have been gained in the determination of moisture content of a batch when the method according to the invention is applied to a concrete mixing process.

More specifically, the method according to the invention is characterized by what is stated in the characterizing part of claim 1.

Next, the invention will be described in greater detail by making reference to the annexed drawing in which

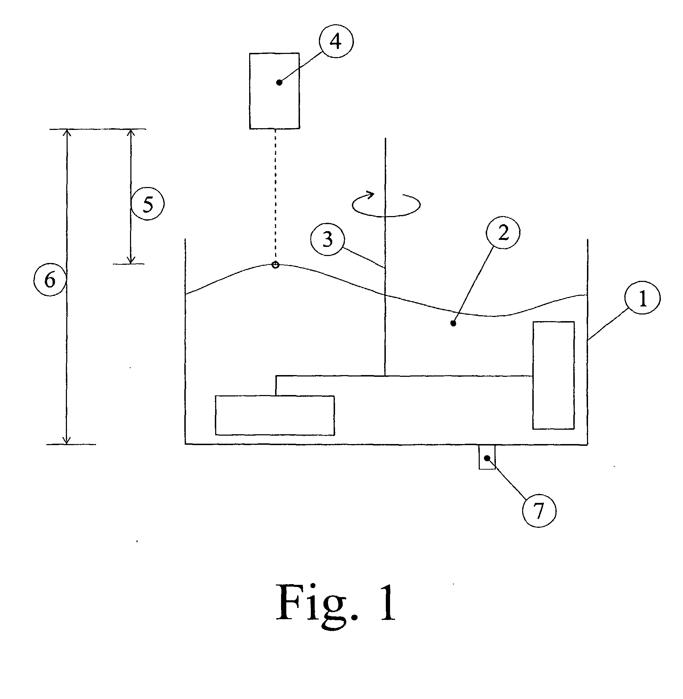

FIG. 1 shows a measurement system arrangement according to the invention.

Referring to FIG. 1, therein is schematically outlined a concrete mixing vessel 1, batch 2 being mixed in the vessel, mixer means 3 and a concrete mix level gauging device 4. The device 4 is adapted to measure distance 5 to the top level of the batch. Inasmuch as distance 6 to the bottom of the vessel as well as the vessel shape are known, the batch volume in the vessel and, thus the density of the batch, can be determined. In a practical system, it is sufficient to get information on changes in the batch level.

A calibration function separately for each aggregate mix recipe and mixing system is generated by sampling different batches and measuring the moisture content of the samples, e.g., by drying the samples. Then, by recording the readings of the level gauge and the microwave measurement device during sampling and combining the readings with the moisture content measured from the sample, it is possible to determine the regression function for the actual moisture content. The regression function and its terms may be linear or nonlinear.

In the measurement system layout, a microwave sensor 7 is mounted in an opening made on the wall of the mixer vessel so that the emitting surface of the sensor faces the concrete mix. In moisture content measurements performed in concrete production by means of microwave sensors, the measurement method is based on the attenuation of microwave radiation reflected from the concrete mix back to the sensor and/or a frequency shift detected in the reflected radiation. The measurement is carried out over a given period of time and the continuous measurement signal is sampled over suitable intervals or an average value thereof that are/is selected to optimally represent the properties of the mix being gauged.

The concrete mix level gauge is also arranged to give continuous data on the level of concrete mix in the mixer vessel. The level gauge output signal is processed to remove deviations of the level reading due the action of the mixer elements in the mixer vessel. Then, the conditioned output data allows the actual level of the concrete mix surface to be determined by averaging, for instance, whereby also other random variations are filtered away. The duration of the measurement cycle may be set, e.g., to 10-20 seconds depending on the mixer construction.

By virtue of the above-described measurement arrangement according to the invention, a concrete mix of desired quality can be produced immediately after the calibration of the measurement system.

Obviously, the same disturbing factors as those affecting microwave measurements are also inflicted on moisture content measurement techniques based on sensing electrical parameters such as changes in capacitance and conductivity. Hence, the accuracy of electrical moisture content measurement techniques may also in this case be improved by virtue of complementing these techniques with the present moisture content improvement method according to the invention based on mixer vessel level gauging.

## Claims

1. A method for improving the accuracy of granular material (2) moisture content measurements based on electromagnetic field measurement techniques, **characterized in that** during the measurement by electromagnetic fields, using a microwave sensor (7) mounted in an opening in a mixer vessel (1), the level of the granular material (2) in said mixer vessel (1) is gauged during mixing and the level gauge output signal is processed to remove deviations of the level reading due to the action of mixer elements (3) in the mixer vessel (1), and based on the gauging result, measurement error caused by variations in bulk density of said granular material (2) is compensated by means of a known parameter dependence function based on the bulking effect, whereby in addition to gauging the level of said granular material (2) the measurement accuracy is improved by using other correction factors affecting the change of dielectric property of the granular material (2) known or measured at the instant of said granular material (2) being mixed.

2. The method of claim 1, **characterized in that** the level of the granular material (2) in the mixer vessel (1) is gauged using a level gauge (4) based on ultrasonic, laser or the like gauging technique.

3. The method of any of the preceding claims , **characterized in that** the material whose moisture content is measured is a concrete mix.

## Patentansprüche

1. Verfahren zur Verbesserung der Genauigkeit der Messung des Feuchtigkeitsgehalts von granuliertem Material (2) mittels der auf Nutzung von elektromagnetischen Feldern basierenden Meßtechnologie, **dadurch gekennzeichnet, dass** während des Messens mittels elektromagnetischer Felder unter Verwendung eines in eine Öffnung eines Mischgefäßes (1) montierten Mikrowellensensors (7) der Füllstand des in dem genannten Mischgefäß (1) befindlichen granulierten Materials (2) während des Mischens gemessen wird, und der Ausgangssignal des gemessenen Füllstands verarbeitet wird, um Abweichungen des Füllstandmeßwerts, die durch Wirkung der in dem Mischgefäß (1) befindlichen Mischelemente (3) entstehen, zu eliminieren, und dass aufgrund des Meßergebnisses Meßfehler, die infolge von Variationen der Schüttdichte des granulierten Materials (2) entstehen, mittels einer bekannten, auf Feuchtigkeitsausdehnung basierenden parameterabhängigen Funktion kompensiert werden, wobei die Genauigkeit der Messung zusätzlich zu dem Messen des Füllstandes des granulierten Materials (2) durch Verwendung anderer Korrekturfaktoren, die eine Veränderung der bekannten oder zur Zeit des Mischens des granulierten Materials (2) gemessenen dielektrischen Eigenschaft des granulierten Materials (2) bewirken, verbessert wird.

2. Verfahren nach Anspruch 1, **dadurch gekennzeichnet, dass** der Füllstand des in dem Mischgefäß (1) befindlichen granulierten Materials (2) durch Verwendung eines auf Ültraschall-, Laser- oder ähnlicher -meßtechnik basierenden Füllstandmeßgeräts (4) gemessen wird.

3. Verfahren nach einem der vorangehenden Ansprüche, **dadurch gekennzeichnet, dass** das Material, dessen Feuchtigkeitsgehalt gemessen wird, eine Betonmischung ist.

## Revendications

1. Procédé destiné à améliorer la précision de mesures de teneur en humidité de matériau granulaire (2) sur la base de procédés de mesure de champ électromagnétique, **caractérisé en ce que**, au cours de la mesure par des champs électromagnétiques, en utilisant un capteur à micro-ondes (7) monté sur une ouverture d'une cuve de mélangeur (1), le niveau du matériau granulaire (2) dans ladite cuve de mélangeur (1) est mesuré au cours du mélange et le signal de sortie d'indicateur de niveau est traité afin d'éliminer des écarts sur la lecture de niveau dus à l'action d'éléments de mélangeur (3) dans la cuve de mélangeur (1), et, sur la base du résultat de mesure, l'erreur de mesure provoquée par des variations sur la densité brute dudit matériau granulaire (2) est compensée au moyen d'une fonction de dépendance de paramètre connue, basée sur l'effet de gonflement, de telle sorte que, en plus de la mesure du niveau dudit matériau granulaire (2) la précision de mesure est améliorée en utilisant d'autres facteurs de correction affectant la variation de propriété diélectrique du matériau granulaire (2) connue ou mesurée à l'instant où ledit matériau granulaire (2) est mélangé.

2. Procédé selon la revendication 1, **caractérisé en ce que** le niveau du matériau granulaire (2) dans la cuve de mélangeur (1) est mesuré à l'aide d'un indicateur de niveau (4) sur la base d'un procédé de mesure par ultrasons, laser ou analogue.

3. Procédé selon l'une quelconque des revendications précédentes, **caractérisé en ce que** le matériau dont la teneur en humidité est mesurée est un mélange de béton.

EP 1 308 725 B1

Fig. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 21359138 A

- US 5025954 A